# FASCICULE DE BREVET EUROPEEN

(11) **EP 3 848 076 B1**
(45) Date de publication et mention de la délivrance du brevet: **12.10.2022**
(21) Numéro de dépôt: 20216719.3
(22) Date de dépôt: 22.12.2020
(51) Int. Cl.: A61M 16/00, A61M 16/10

(54) **ENSEMBLE DE FOURNITURE DE GAZ COMPRENANT UN RÉCIPIENT DE GAZ**
GASVERSORGUNGSANORDNUNG MIT EINEM GASBEHÄLTER
GAS SUPPLY ASSEMBLY COMPRISING A GAS CONTAINER

(30) Priorité: 07.01.2020 FR 2000092
(43) Date de publication de la demande: 14.07.2021
(73) Titulaire: L'Air Liquide, société anonyme pour l'Étude et l'Exploitation des procédés Georges Claude, 75007 Paris (FR)
(72) Inventeur: BOULANGER, Thierry, Newark, DE 19702 (US)
(74) Mandataire: Air Liquide

(56) Documents cités:
- US-A1- 2012 097 879
- US-A1- 2017 043 318
- US-B2- 8 894 751

## Description

La présente invention concerne un ensemble de fourniture de gaz comprenant un récipient de gaz et un système de soutirage de gaz, coopérant avec le récipient de gaz, pour assurer une délivrance de gaz, en particulier d'oxygène (O₂) à haute concentration d'O₂ à un utilisateur, tel un patient.

L'administration d'O₂ gazeux sert à corriger une situation hypoxémique, c'est-à-dire lorsque la saturation d'oxyhémoglobine dans le sang devient ou est inférieure à une valeur normale, par exemple inférieure à 90%, chez différentes populations, i.e. nouveaux nés, enfants ou adultes, souffrant de différentes pathologies, par exemple Bronchopneumopathie Chronique Obstructive (BPCO), Syndrome de Détresse Respiratoire Aigüe (SDRA)...

A l'hôpital, l'O₂ est usuellement délivré via des prises murales, connectées fluidiquement à une source d'O₂ afin d'assurer un apport continu d'O₂ aux patients.

Hors des hôpitaux, par exemple dans les unités mobiles, telles que les ambulances, ou au domicile des patients, on a recours à des bouteilles de gaz contenant de l'O₂ de qualité médicale comprimé à une pression allant jusqu'à environ 200 bar abs. De telles bouteilles peuvent être transportées et maniées par une seule personne. Ainsi, les bouteilles appelées « B2 » et « B5 » renferment respectivement 2 et 5 L de gaz à 200 bars, soit des volumes de gaz de 400 et 1000 L (mesurés à 1 atm). Le débit d'oxygène délivré par de telles bouteilles est réglé selon l'indication, avec, dans les cas les plus aigus, des débits de l'ordre de 15 L/min pour garantir une haute concentration d'oxygène inhalé. C'est le cas par exemple en situation d'urgence, lors d'une noyade ou d'une intoxication au monoxyde de carbone (CO), mais aussi au domicile de patients souffrant d'algie vasculaire de la face.

A un tel débit, une bouteille B2 garantit environ 25 min de thérapie, ce qui serait suffisant dans un certain nombre de situations, notamment dans l'attente de premiers secours ou le temps que la crise d'algie vasculaire de la face ne s'estompe, mais malheureusement impossible à mettre en pratique du fait de contraintes inhérentes à la technologie. Par exemple, les bouteilles d'oxygène sont lourdes et encombrantes, donc difficilement transportable pour un patient souhaitant s'éloigner de son domicile. Ainsi, une bouteille B2 pèse environ 6kg pour une hauteur de 50 cm environ.
La pression importante régnant dans les bouteilles limite par ailleurs les lieux dans lesquels de telles sources d'oxygène peuvent être accessibles, notamment les lieux publics où des premiers soins à une personne en détresse pourraient être prodigués.

Une alternative à ces limitations pourrait être d'utiliser un concentrateur d'oxygène portatif comme proposé par US-A-8,894,751, qui permet de produire un flux gazeux riche en oxygène à partir d'air ambient aspiré et comprimé, puis soumis à une séparation par adsorption de l'azote sur un tamis moléculaire, l'adsorbant étant contenu dans une enceinte alimentée en air ambient par un compresseur externe. La principale limitation de ce type de dispositif est qu'on ne peut produire et fournir qu'une quantité faible d'oxygène, typiquement 1 à 3 L/min. Cela ne répond donc pas aux besoins d'inhalation d'oxygène à hautes concentrations. Par ailleurs, du fait du prélèvement d'air ambient pour en extraire l'oxygène, un tel dispositif ne peut être utilisé dans les lieux où l'air ambient est lui-même contaminé, par exemple par du monoxyde de carbone (CO).

En d'autres termes, des applications nécessitant l'application d'une haute concentration d'oxygène pendant une durée de plusieurs dizaines de minutes se trouvent actuellement sans solution technique capable de répondre aux contraintes réglementaires d'entreposage, du fait d'une pression trop importante, et par ailleurs de maniabilité et d'encombrement, i.e. poids, volume...

Le problème est de proposer un ensemble de fourniture de gaz permettant de fournir de l'oxygène (O₂) à haute concentration d'O₂ à un utilisateur, tel un patient, et donc répondre à l'ensemble des limitations et/ou inconvénients susmentionnés de manière à faciliter l'usage d'O₂ et d'étendre son champ d'application, en particulier lorsque de l'O₂ à haute concentration est requis, comme par exemple en cas d'empoisonnement au CO, de noyade en piscine, de crise d'algie vasculaire de la face ou autre.

Une solution de l'invention concerne alors un ensemble de fourniture de gaz, en particulier d'oxygène, comprenant un récipient de gaz et un système de soutirage de gaz coopérant avec le récipient de gaz, dans lequel :
- le récipient de gaz comprend une enveloppe périphérique définissant un volume interne contenant au moins un matériau adsorbant, et
- le système de soutirage de gaz comprend :
   i) un mécanisme d'amorçage mobile comprenant des moyens de perçage,
   ii) et un mécanisme d'activation actionnable par un utilisateur et coopérant avec le mécanisme d'amorçage pour déplacer le mécanisme d'amorçage en direction du récipient de gaz de manière à ce que les moyens de perçage du mécanisme d'amorçage viennent perforer, i.e. percer, l'enveloppe périphérique du récipient de gaz.

Selon l'invention, le mécanisme d'activation du système de soutirage de gaz comprend :
- un corps principal dans lequel est agencé le mécanisme d'amorçage, ledit corps principal comprenant un premier et un second orifice, et
- un percuteur mobile comprenant une première et une deuxième chambre de percuteur comprenant chacune un orifice d'entrée/sortie de gaz, ledit percuteur comprenant un prolongement de paroi configuré pour :
   i) obturer le second orifice du corps principal du mécanisme d'activation, avant perçage de l'enveloppe périphérique du récipient de gaz par les moyens de perçage du mécanisme d'amorçage, et
   ii) libérer le second orifice du corps principal du mécanisme d'activation, après perçage de l'enveloppe périphérique du récipient de gaz par les moyens de perçage du mécanisme d'amorçage et mettre le second orifice du corps principal en communication fluidique avec la première chambre de percuteur via l'orifice d'entrée/sortie de gaz de ladite première chambre de percuteur.

Selon le mode de réalisation considéré, l'ensemble de fourniture de gaz de l'invention peut comprendre l'une ou plusieurs des caractéristiques suivantes :
- les moyens de perçage comprennent des pointes creuses, c'est-à-dire comprenant un lumen ou canal interne de passage de gaz.
- le récipient de gaz est une cartouche de gaz, notamment à usage unique, en particulier d'oxygène.
- le système de soutirage de gaz est agencé et fixé sur le récipient de gaz
- une paroi de séparation est agencée axialement dans le volume interne de l'enveloppe périphérique et divise ledit volume interne en un premier et un second compartiment, les premier et second compartiments contenant au moins ledit matériau adsorbant.
- la paroi de séparation comprend au moins un passage de gaz mettant en communication fluidique les premier et second compartiments.
- les premier et second compartiments sont agencés de manière symétrique par rapport à la paroi de séparation.
- les premier et second compartiments sont identiques, c'est-à-dire sensiblement de même volume.
- la paroi de séparation comprend un bord périphérique venant se raccorder de manière étanche à la paroi interne de l'enveloppe périphérique du récipient.
- chacun des premier et second compartiments comprend un lit de matériau adsorbant et une chambre de recueil de gaz.
- chaque chambre de recueil de gaz surmonte un lit de matériau adsorbant.
- l'adsorbant comprend une zéolite.
- un filtre poreux est agencé dans chacun des premier et second compartiments entre le lit de matériau adsorbant et la chambre de recueil de gaz.
- l'enveloppe périphérique est en métal, par exemple en alliage d'aluminium.
- l'enveloppe périphérique a une forme générale cylindrique.
- l'enveloppe périphérique comprend un fond et un couvercle.
- le corps d'enveloppe de forme générale cylindrique s'étend entre le fond et le couvercle.
- l'enveloppe périphérique comprend une valve de remplissage permettant de remplir le volume interne avec un gaz, tel de l'oxygène.
- les chambres de recueil de gaz sont agencées immédiatement sous le couvercle de l'enveloppe périphérique.
- le mécanisme d'activation coopère avec le mécanisme d'amorçage pour déplacer les pointes creuses en rapprochement (ou en éloignement) par rapport au récipient de gaz.
- le mécanisme d'amorçage comprend une première et une deuxième chambre de percuteur comprenant chacune un orifice de sortie de gaz.
- les pointes creuses comprenant des extrémités libres acérées et sont en communication fluidique avec les chambres de percuteur, via un canal interne.
- le mécanisme d'amorçage comprend une pointe creuse additionnelle ayant une longueur supérieure à la longueur des pointes creuses.
- le mécanisme d'activation du système de soutirage de gaz comprend un corps principal dans lequel est agencé le mécanisme d'amorçage.
- le corps principal forme manchon autour d'une partie du récipient.
- le mécanisme d'amorçage comprend le percuteur mobile.
- les première et deuxième chambres de percuteur sont séparées par une cloison de séparation.
- les canaux internes des pointes creuses sont en communication fluidique avec les première et deuxième chambre de percuteur.
- les canaux internes des pointes creuses relient fluidiquement les première et deuxième chambre de percuteur aux chambres de recueil de gaz du récipient.
- les pointes creuses des moyens de perçage comprennent au moins une pointe creuse additionnelle ayant une longueur supérieure à la longueur de la majorité des autres pointes creuses, ladite pointe creuse additionnelle étant en communication fluidique avec la deuxième chambre de percuteur, via son canal interne.
- les pointes creuses des moyens de perçage sont portées par des éléments tubulaires longilignes, tel que des tubulures creuses ou analogues, en particulier les pointes creuses sont agencées à une extrémité libre desdits éléments tubulaires longilignes.
- le mécanisme d'activation comprend un organe de manipulation, par exemple une manivelle, un volant rotatif, un levier pivotant ou analogue, actionnable par l'utilisateur coopérant avec une tige filetée venant appuyer sur le percuteur mobile du mécanisme d'amorçage.
- le volume interne du récipient de gaz contient de l'oxygène à une pression inférieure ou égale à 10 bar absolus.
- après perçage de l'enveloppe périphérique du récipient de gaz par les moyens de perçage du mécanisme d'amorçage, le premier orifice du corps principal est en communication fluidique avec la deuxième chambre de percuteur via l'orifice d'entrée/sortie de gaz de ladite deuxième chambre de percuteur.
- le second orifice du corps principal du mécanisme d'activation est en communication fluidique avec une interface respiratoire, en particulier un masque naso-buccal, i.e. facial.
- le premier orifice du corps principal est en communication fluidique avec un réservoir flexible.
- après perçage de l'enveloppe périphérique du récipient de gaz par les moyens de perçage du mécanisme d'amorçage :
   ▪ le premier orifice du corps principal est en communication fluidique avec la deuxième chambre de percuteur via l'orifice d'entrée/sortie de gaz de ladite deuxième chambre de percuteur.
   ▪ et/ou le premier orifice du corps principal du mécanisme d'activation est en communication fluidique avec le réservoir flexible qui se remplit avec de l'oxygène libéré de l'enveloppe périphérique du récipient de gaz.
   ▪ et/ou le second orifice du corps principal est en communication fluidique avec la première chambre de percuteur via l'orifice d'entrée/sortie de gaz de ladite première chambre de percuteur.
   ▪ et/ou le second orifice du corps principal du mécanisme d'activation est en communication fluidique avec une interface respiratoire à laquelle un patient est connecté.
   ▪ et/ou les premier et second orifices du corps principal du mécanisme d'activation sont en communication fluidique au travers des seconde et première chambres de percuteur et de l'enveloppe périphérique du récipient de gaz.
   ▪ et/ou les premier et second orifices du corps principal du mécanisme d'activation sont en communication fluidique au travers des seconde et première chambres de percuteur et de l'enveloppe périphérique du récipient de gaz.
   ▪ et/ou le réservoir flexible fournit au patient de l'oxygène haute concentration lors d'une inhalation dudit patient, c'est-à-dire une concentration d'O₂ d'au moins 90 % vol, en particulier d'au moins 95 % vol., voire plus.
   ▪ et/ou l'enveloppe périphérique du récipient de gaz capture le CO₂ expiré par le patient lorsque les gaz expirés empruntent un chemin retour allant de l'interface respiratoire vers le réservoir flexible.

En outre, l'invention concerne aussi l'utilisation d'un ensemble de fourniture de gaz selon l'invention pour stocker ou pour fournir un gaz à un utilisateur, en particulier de l'oxygène à un patient.

L'invention va maintenant être mieux comprise grâce à la description détaillée suivante, faite à titre illustratif mais non limitatif, en référence aux figures annexées parmi lesquelles :
Fig. 1 est une vue schématique en coupe d'un mode de réalisation d'un récipient 1 de stockage de gaz utilisable dans un système de soutirage de gaz selon l'invention,
Fig. 2 représente un mode de réalisation de la paroi de séparation axiale du récipient de Fig. 1,
Fig. 3 représente un mode de réalisation d'un système de soutirage de gaz pour un système de soutirage de gaz selon l'invention,
Fig. 4 à Fig. 8 schématisent un mode de réalisation d'un ensemble de fourniture de gaz selon l'invention comprenant le système de soutirage de gaz de Fig. 3 et le récipient de stockage de Fig. 1.

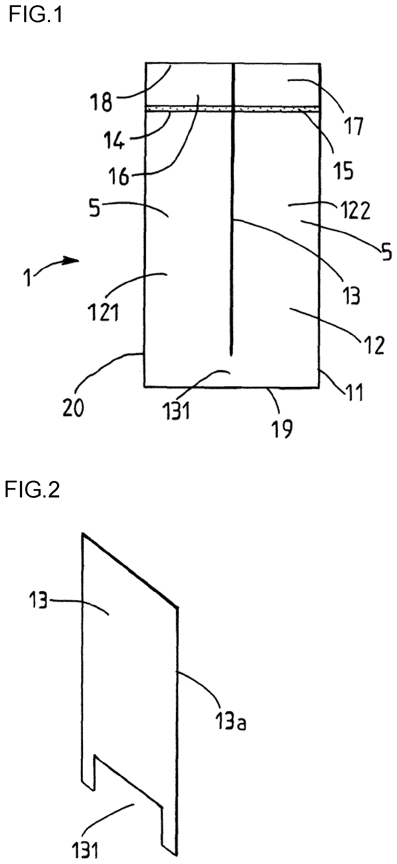

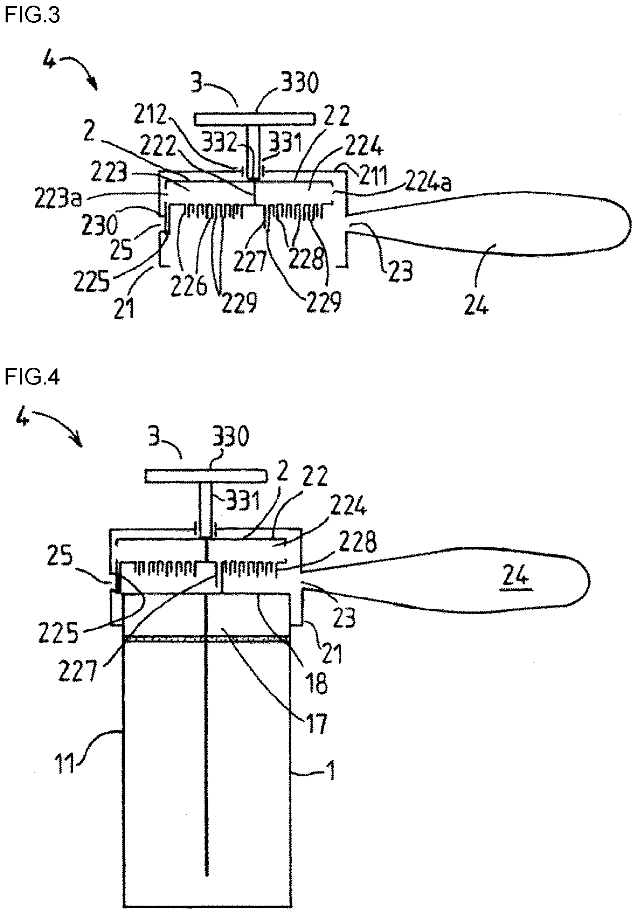

Fig. 1 est une vue schématique en coupe d'un mode de réalisation d'un récipient de gaz 1, telle une cartouche de gaz à usage unique, utilisable dans un ensemble de fourniture de gaz 1, 4 selon l'invention utilisable dans l'ensemble de fourniture de gaz selon l'invention illustré sur Fig. 4 à Fig. 8.

Le récipient de gaz 1 de Fig. 1 comprend une enveloppe périphérique 11 externe, de préférence en métal, par exemple un alliage d'aluminium, définissant un volume interne 12 servant au stockage de gaz, en particulier d'oxygène (O₂).

De préférence, l'enveloppe périphérique 11 externe du récipient de gaz 1 comprend un corps allongé de forme cylindrique 20, un fond 19 et un couvercle ou « toit » 18 séparés l'un de l'autre par le corps cylindrique 20. Le fond 19 et le couvercle 18 sont fixés de manière étanche, par exemple soudés ou sertis, au corps cylindrique 20. La paroi de l'enveloppe périphérique 11 est fine, c'est-à-dire a une épaisseur de l'ordre de quelques dixièmes de mm.

Le volume intérieur 12 de l'enveloppe 11 comprenant au moins un (i.e. un ou plusieurs) adsorbant 5 agencé dans deux compartiments parallèles 121, 122 séparés par une paroi axiale 13 agencée axialement et diamétralement dans le volume interne 12 de l'enveloppe 11. La paroi axiale 13 permet de diviser le volume interne 12 des deux compartiments 121, 122, c'est-à-dire deux sous-volumes, distincts et préférentiellement égaux, c'est-à-dire présentant sensiblement un même volume. Les compartiments 121, 122 sont partiellement remplis d'adsorbant 5.

Comme montré sur Fig. 2, la paroi axiale 13, vue en perspective, comprend un rebord périphérique 13a ou périphérie venant se raccorder à la paroi interne de l'enveloppe 11 du récipient 1, par exemple par soudage ou analogue, à savoir au fond 19, au couvercle 18 et au corps cylindrique 20.

L'adsorbant 5 peut être du charbon actif, un MOF (« Metal Organic Framework ») ou de la zéolite, servant à adsorber des molécules de gaz, en particulier de l'oxygène, du fait de minuscules pores à leur surface (de l'ordre du nanomètre). De préférence, on utilise un adsorbant zéolitique ayant une capacité d'adsorption élevée, par exemple une zéolite de type ZEOCHEM Z10-ZZ ou 5A sous forme de granules ou de billes.

Comme visible sur Fig. 1, l'adsorbant 5 ne remplit pas l'intégralité de chacun des compartiments 121, 122 de l'enveloppe 11 de sorte qu'il existe un espacement formant une chambre de recueil de gaz 16, 17 surmontant chaque lit d'adsorbant 5 au sein de chacun des compartiments 121, 122.

De préférence, deux filtres poreux 14, 15 sont agencés entre chaque lit d'adsorbant 5 et chaque chambre de recueil de gaz 16, 17, c'est-à-dire que les filtres poreux 14, 15 surmontent l'adsorbant 5 de manière à le circonscrire et/ou le maintenir dans chacun des compartiments 121 et 122. Les filtres 14, 15 poreux autorisent une circulation des molécules de gaz tout en empêchant d'éventuelles poussières libérées par les particules d'adsorbant 5 de migrer dans les chambres de recueil de gaz 16, 17. Les filtres 14, 15 peuvent par exemple se présenter sous formes de mousses comprenant ou formées d'alvéoles interconnectées.

La paroi de séparation axiale 13 comprend un passage de gaz ou évidement 131 a été réalisé en partie basse de la paroi axiale 13 de manière à assurer un passage de gaz, c'est-à-dire une communication fluidique, entre les lits d'adsorbant 5 situés dans les deux compartiments 121, 122, au niveau du fond 19 de l'enveloppe 11.

Avant utilisation, le récipient 1, telle une cartouche, est rempli d'O₂ à une pression de quelques bars, par exemple au maximum de l'ordre de 10 bars absolus, ce qui permet d'y stocker une quantité d'oxygène très supérieure à celle que pourrait contenir le récipient 1 en l'absence d'adsorbant, et par ailleurs de répondre aux contraintes de logistiques, notamment d'acheminement, et d'entreposage. L'introduction de l'O₂ dans l'enveloppe 11 peut se faire via une valve de remplissage (non montrée) portée par l'enveloppe 11, par exemple agencée sur le couvercle 18 ou ailleurs. L'adsorbant 5, de préférence une zéolite, permet donc d'augmenter très notablement la capacité de stockage du gaz au sein de l'enveloppe 11.

Ainsi, à une pression de 10 bars, une cartouche 1 de 660 mL de volume interne 12 peut contenir environ 330 g d'adsorbant 5 et, par conséquent, adsorber environ 12 L d'O₂, alors que l'espace interstitiel autour des particules d'adsorbant 5 définit un volume « vide » dans lequel des molécules d'O₂ vont également être comprimées et stockées, permettant ainsi d'augmenter la capacité totale à environ 15 L d'O₂ (à pression atmosphérique). A titre comparatif, en l'absence d'adsorbant 5, on ne pourrait y stocker qu'environ 6 L d'O₂.

Fig. 3 est une vue en coupe d'un mode de réalisation d'un système de soutirage de gaz 4 configuré pour coopérer avec le récipient 1 de la Fig. 1 afin d'en extraire le gaz, tel de l'O₂, qui y est stocké, en formant ainsi un ensemble de fourniture de gaz selon l'invention, comme illustré sur Fig. 4 à Fig. 8.

Le système de soutirage de gaz 4 de Fig. 3 comprend un mécanisme d'amorçage 2 et un mécanisme d'activation 3 coopérant l'un avec l'autre. Il est conçu pour venir se positionner et se fixer ici sur le haut du récipient 1 de l'invention, c'est-à-dire au niveau de son couvercle 18, comme illustré sur les Fig. 4 à Fig. 8.

Plus précisément, le système de soutirage de gaz 4 comprend un corps principal 21 ayant une forme générale de « coupelle » fermée à son extrémité supérieure par un fond borgne 211, et formant un manchon autour de la partie supérieure de l'enveloppe 11 du récipient 1, c'est-à-dire autour du haut du récipient 1 de l'invention, lorsque le système de soutirage de gaz est couplé au récipient 1, comme illustré sur Fig. 4 à Fig. 8.

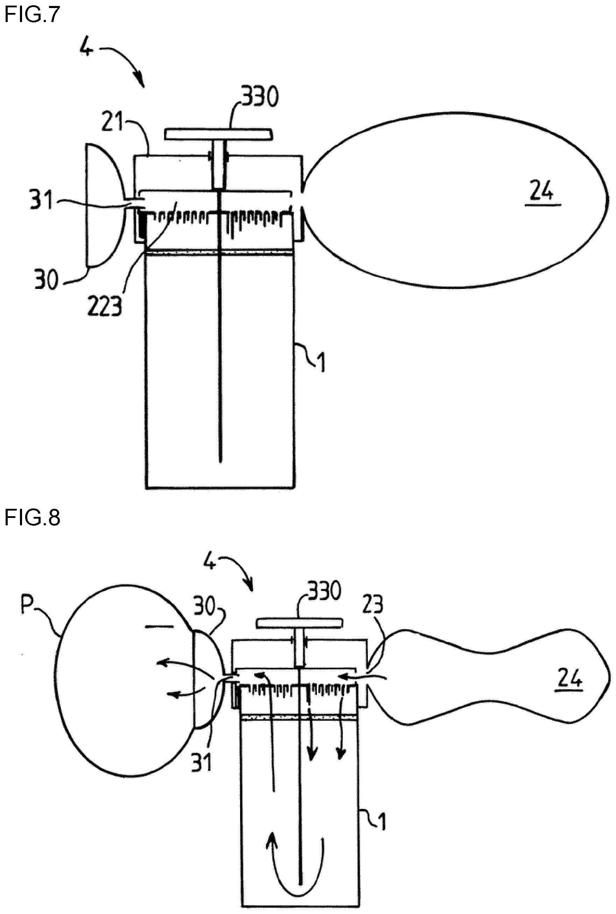

Le corps principal 21 du système de soutirage de gaz 4 comporte deux orifices 25, 23, de préférence cylindriques, aménagés dans sa paroi latérale, comme illustré en Fig. 3, de préférence des orifices 25, 23 diamétralement opposés. Le premier orifice 23, situé latéralement dans le corps principal 21, débouche dans un réservoir déformable 24 servant à récupérer le gaz extrait du récipient 1. Le second orifice 25 sert à alimenter une interface respiratoire 30, comme expliqué ci-après et visible sur Fig. 7.

Le mécanisme d'amorçage 2 est agencé dans le corps principal 21 du système de soutirage de gaz 4, alors que le mécanisme d'activation 3 coopérant avec le mécanisme d'amorçage 2 est, quant à lui, agencé principalement à l'extérieur du corps principal 21 du système de soutirage de gaz 4 mais traverse le fond borgne 211 dudit corps principal 21 du système de soutirage de gaz 4 de sorte à pouvoir être actionné manuellement par l'utilisateur et venir alors coopérer avec le mécanisme d'amorçage 2 en venant appuyer mécaniquement sur celui-ci, comme expliqué ci-après.

Le mécanisme d'amorçage 2 comprend un percuteur 22 comprenant deux chambres de percuteur 223, 224, à savoir une première 223 et une deuxième 224 chambre de percuteur, agencées en parallèle l'une de l'autre et séparées par une cloison de séparation 222 étanche. Les deux chambres de percuteur 223, 224 comprennent chacune un orifice d'entrée/sortie de gaz 223a, 224a.

Le mécanisme d'amorçage 2 comprend en outre un prolongement de paroi 225 venant obstruer le second orifice 25 avant utilisation de l'ensemble de fourniture de gaz 4 de l'invention. Pour assurer une meilleure étanchéité, un joint d'étanchéité 230, par exemple un joint torique, est agencé entre le prolongement de paroi 225 et la périphérie du second orifice 25.

Le mécanisme d'amorçage 2 comprend aussi des moyens de perçage 226-228 incluant une pluralité de pointes creuses 226, 228 comprenant des extrémités libres acérées, par exemple biseautées, lesquelles sont agencées axialement dans le prolongement des chambres de percuteur 223, 224 et en communication fluidique avec l'une ou l'autre des chambres de percuteur 223, 224. Plus précisément, une première série de pointes creuses 226 débouche dans la première chambre de percuteur 223, et une seconde série de pointes creuses 228 débouche dans la seconde chambre de percuteur 224.

Les pointes creuses 228 sont portées par des éléments tubulaires longilignes, tel que des tubulures creuses ou analogues, en particulier les pointes creuses sont agencées à une extrémité libre desdits éléments tubulaires longilignes.

Les pointes creuses 226, 228 présentent donc chacune un canal interne 229 en communication fluidique avec l'une des chambres de percuteur 223, 224.

Par ailleurs, une pointe additionnelle 227 plus longue que les pointes 226, 228, est agencée dans le prolongement de la seconde chambre de percuteur 224, et comprend elle-aussi un canal interne 229 en relation fluidique avec la seconde chambre de percuteur 224, et une extrémité libre acérée.

Les pointes acérées 226, 227, 228 constituent des éléments pointus creux suffisamment solides et résistants pour pouvoir percer le couvercle 18 du récipient 1 de l'invention, sans se briser ou se déformer, par exemple elles sont formées d'acier inoxydable.

Le perçage produit par les pointes acérées 226 et, de même, par les pointes acérées 227,228 conduit à obtenir des surfaces d'échange d'au moins 150 mm², entre la chambre de percuteur et l'intérieur de la cartouche pour permettre au patient de respirer facilement.

Par ailleurs, comme illustré en Fig. 3, le mécanisme d'activation 3 comprend un organe de manipulation 330, tel un levier, une manivelle ou un volant rotatif, coopérant avec une tige filetée 331 mécaniquement couplée à un orifice fileté 212 aménagé dans le fond borgne 211 du corps principal 21 du système de soutirage de gaz 4.

Lorsqu'un utilisateur actionne l'organe de manipulation 330, par exemple selon un mouvement de vissage horaire (ou à l'inverse antihoraire), la tige filetée 331 se déplace au sein de l'orifice fileté 212, en rapprochement (ou en éloignement) par rapport au mécanisme d'amorçage 2.

Comme représenté sur la Figure 3, la tige filetée 331 et le percuteur 22 du mécanisme d'amorçage 2 sont en contact mécanique l'un avec l'autre, via une surface d'appui 332 de sorte que la tige filetée 331 du mécanisme d'activation 3 puisse venir appuyer mécaniquement sur le percuteur 22 du mécanisme d'amorçage 2 pour le repousser en direction du récipient 1 de gaz lorsqu'un utilisateur actionne l'organe de manipulation 330 pour déplacer la tige filetée 331 en direction du mécanisme d'amorçage 2.

Fig. 4 montre le système de soutirage de gaz 4 positionné sur le récipient de gaz 1 de Fig. 1, avant perçage du couvercle 18 du récipient 1 par les pointes acérées 226, 227, 228 pour en soutirer l'oxygène qui y est stocké.

Le corps principal 21 du système de soutirage de gaz 4 est positionné et couplé mécaniquement, i.e. solidarisé, à l'enveloppe 11 de la cartouche 1 de manière à y être retenu en position, par exemple par montage en force, via un filetage ou par tout autre moyen de fixation. Comme on le voit, les pointes 226, 227, 228 n'ont pas encore percé le couvercle 18 du récipient 1 de l'invention. Le second orifice 25 est quant à lui obturé par le prolongement de paroi 225, alors que le premier orifice 23 est en communication fluidique avec le réservoir 24 qui est vide, c'est-à-dire non rempli de gaz.

Lorsqu'un utilisateur actionne l'organe de manipulation 330 du mécanisme d'activation 3, par exemple opère un vissage dans le sens horaire de l'organe de manipulation 330, la tige filetée 331, qui est solidaire dudit organe de manipulation 330, se déplace et vient appuyer sur le percuteur 22 du mécanisme d'amorçage 2 pour le déplacer en rapprochement du couvercle 18 du récipient 1.

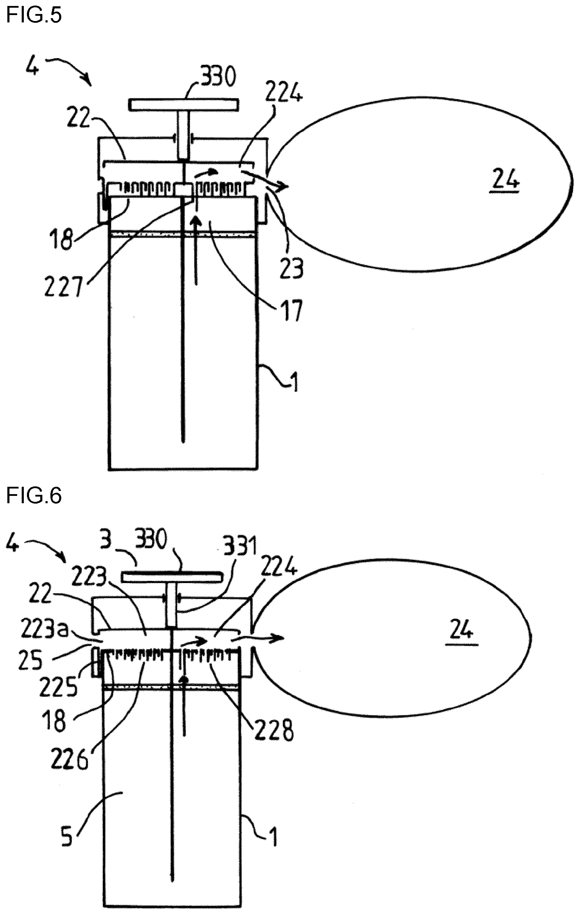

Comme illustré en Fig. 5, il va alors se produire un perçage du couvercle 18 d'abord par la pointe additionnelle 227 qui est plus longue que les séries de pointes 226, 228, ce qui met en communication fluidique la chambre de recueil de gaz 17 du récipient 1 et la seconde chambre 224 du percuteur 22, via le canal interne 229 de la pointe 227.

Il s'opère alors un transfert de gaz, i.e. O₂, depuis l'intérieur du récipient 1 vers la seconde chambre 224 du percuteur 22, puis ensuite de la seconde chambre 224 au réservoir 24 flexible, via le premier orifice de sortie 23. Le flux gazeux sous pression va alors progressivement remplir le réservoir 24 qui augmente en volume.

Par ailleurs, le prolongement de paroi 225 empêche toujours que de l'O₂ ne puisse s'échapper par le second orifice 25 en obturant ledit second orifice 25.

Transpercer le couvercle 18 de l'enveloppe 11 à un seul endroit, via la pointe additionnelle 227 permet de limiter la quantité de gaz s'échappant du récipient 1 afin de limiter le débit de remplissage du réservoir flexible 24 et opérer ainsi une transition plus « douce » du fait du différentiel de pression entre intérieur (10 bar) et extérieur du récipient (1 bar), i.e. dans le réservoir 24.

Comme illustré en Fig. 6, lorsque l'utilisateur continue son actionnement de l'organe de manipulation 330 du mécanisme d'activation 3, la tige filetée 331 continuer à appuyer sur le percuteur 22 du mécanisme d'amorçage 2 et à le déplacer en rapprochement du couvercle 18 du récipient 1 jusqu'à ce que les séries de pointes 226, 228 viennent elles-aussi percer le couvercle 18 du récipient 1 et mettre les chambres de recueil de gaz 16, 17 du récipient 1 en communication fluidique avec les deux chambres de percuteur 223, 224, à savoir la première 223 et la deuxième 224 chambre de percuteur, via les canaux internes 229 des pointes creuses 226, 228. Ceci permet à l'intégralité de l'O₂ présent dans le récipient 1, notamment dans l'adsorbant 5, de sortir du récipient 1 et de notamment migrer vers le réservoir 24 flexible pour le remplir de gaz.

Les ouvertures des canaux internes 229 des séries de pointes 226, 227 du percuteur 22 forment une surface d'échange comprise entre 150 et 700 mm² pour chaque série de pointes, soit de 300 à 1400 mm² au total.

Le mouvement de translation engendré par l'actionnement de l'organe de manipulation 330 par l'utilisateur est stoppé lorsque le percuteur 22 vient en butée contre la surface externe du couvercle 18 de l'enveloppe 11, comme illustré en Fig. 6. On remarque que le prolongement de paroi 225 n'obstrue plus le second orifice 25, ce dernier étant alors situé en regard et en communication fluidique avec le second orifice 223a de la deuxième chambre de percuteur 223.

Le système est alors amorcé et prêt à l'emploi, c'est-à-dire prêt à fournir de l'oxygène à un utilisateur, par exemple un patient. Pour ce faire, on peut raccorder mécaniquement fluidiquement une interface respiratoire 30, tel un masque facial, au niveau du second orifice 25 du corps principal 21, via un conduit 31, comme illustré en Fig. 7. La totalité de l'oxygène sortant de la première chambre 223 est alors dirigée dans le conduit 31 de l'interface respiratoire 30 et l'oxygène peut alors être inhalé par le patient P, pendant plusieurs minutes, voire plusieurs dizaines de minutes.

Comme illustré en Fig. 8, lors des phases inhalatoires du patient, une partie de l'O₂ contenu dans le réservoir flexible 24, ressort de celui-ci via son orifice 23, puis transite successivement dans la seconde chambre de percuteur 224, les canaux internes 229 des pointes 228, 227, la seconde chambre de recueil de gaz 17 de l'enveloppe 11 du récipient 1, l'adsorbant 5, la première chambre de recueil de gaz 16 de l'enveloppe 11 du récipient 1, les canaux internes 229 des pointes 226 et la première chambre de percuteur 223, avant de pénétrer dans le conduit 31 de l'interface respiratoire 30 et enfin le volume interne de l'interface 30 pour être inhalé par le patient P.

La multitude de canaux internes 229 des pointes 226, 227 et 228, en présentant une surface de passage importante, limite l'effort inspiratoire du patient P pour puiser l'O₂ dans le réservoir 24.

Lors des phases expiratoires du patient P, une partie de l'O₂ inhalé (env. 5% vol.) a été substituée par du CO₂ et le gaz exhalé par le patient contient donc du CO₂, de la vapeur d'eau et une haute concentration d'O₂, c'est-à-dire plus de 90 % en volume. Le gaz expiré va suivre un chemin inverse à celui suivi par l'oxygène (i.e. comme illustré en Fig. 8), à savoir quitter l'interface 30 pour entrer dans la première chambre de percuteur 223, emprunter les canaux internes 229 des pointes 226 et en sortir dans la première chambre de recueil de gaz 16, puis transiter dans le récipient 1 au travers de l'adsorbant 5, y compris les filtres poreux 14, 15, et la seconde chambre de recueil de gaz 17, puis en ressortir via les canaux internes 229 des pointes 227, 228, transiter par la seconde chambre de percuteur 224 avant de retourner et remplir le réservoir flexible 24.

Une caractéristique de l'adsorbant 5, en particulier de type zéolites, est que leur capacité d'adsorption diffère selon les molécules concernées. En fait, la capacité d'adsorption de l'O₂ est relativement faible au regard de la capacité d'adsorption du CO₂ ou de la vapeur d'eau, rapportée de 50 à 100 fois plus importante. Y compris à une concentration de 5% de CO₂, c'est-à-dire 5 kPa en pression absolue, la capacité d'adsorption d'une zéolite, par exemple type Zeochem Z10-ZZ ou bien 5A, excède aisément 1.5 moles par kg, présentant ainsi une capacité de stockage en volume supérieure au volume total d'O₂ initialement présent dans la cartouche 1, à 10 bars. Cette capacité est encore plus importante concernant la vapeur d'eau.

Ainsi, lorsque le gaz expiré par le patient traverse l'adsorbant 5, les molécules de CO₂ et la vapeur d'eau vont être adsorbées de sorte que le gaz est débarrassé de la quasi-totalité du CO₂ qu'il contient. Ce gaz exempt de CO₂ peut ensuite être inspiré à nouveau, lors d'une phase inspiratoire suivante.

D'une manière générale, la consommation métabolique en oxygène d'un individu est au maximum de 0.5 L/min, ce qui implique qu'après 10 minutes d'inhalations/expirations répétées, le réservoir 1 contient environ 5L d'O₂ en moins, dans les conditions susmentionnées (pression de 10 bar, volume du récipient de 660 mL). L'ensemble de délivrance d'oxygène de l'invention permet donc de subvenir au besoin d'une thérapie à haute concentration d'O₂ pendant plusieurs dizaines de minutes, et ce, malgré son encombrement réduit.

Bien entendu, d'autres modes de réalisation peuvent être envisagés, par exemple l'utilisation de plusieurs adsorbants différents, par exemple plusieurs lits successifs de différents adsorbants, une perforation différente de l'enveloppe de la cartouche 1, par exemple de son couvercle et de son fond, et/ou par percussion, compression ou autre, une enveloppe ayant une forme différente....

## Revendications

1. Ensemble de fourniture de gaz (1, 4) comprenant un récipient de gaz (1) et un système de soutirage de gaz (4) coopérant avec le récipient de gaz (1), dans lequel :
- le récipient de gaz (1) comprend une enveloppe périphérique (11) définissant un volume interne (12) contenant au moins un matériau adsorbant (5), et
- le système de soutirage de gaz (4) comprend :
a) un mécanisme d'amorçage (2) mobile comprenant des moyens de perçage (226, 227, 228), et
b) un mécanisme d'activation (3) actionnable par un utilisateur et coopérant le mécanisme d'amorçage (2) pour déplacer le mécanisme d'amorçage (2) en direction du récipient de gaz (1) de manière à ce que les moyens de perçage (226, 227, 228) du mécanisme d'amorçage (2) viennent perforer l'enveloppe périphérique (11) du récipient de gaz (1),
**caractérisé en ce que** le mécanisme d'activation (3) du système de soutirage de gaz (4) comprend :
- un corps principal (21) dans lequel est agencé le mécanisme d'amorçage (2), ledit corps principal (21) comprenant un premier (23) et un second orifice (25), et
- un percuteur (22) mobile comprenant une première et une deuxième chambre de percuteur (223, 224) comprenant chacune un orifice d'entrée/sortie de gaz (223a, 224a), ledit percuteur (22) comprenant un prolongement de paroi (225) configuré pour :
1) obturer le second orifice (25) du corps principal (21) du mécanisme d'activation (3), avant perçage de l'enveloppe périphérique (11) du récipient de gaz (1) par les moyens de perçage (226, 227, 228) du mécanisme d'amorçage (2), et
2) libérer le second orifice (25) du corps principal (21) du mécanisme d'activation (3), après perçage de l'enveloppe périphérique (11) du récipient de gaz (1) par les moyens de perçage (226, 227, 228) du mécanisme d'amorçage (2) et mettre le second orifice (25) du corps principal (21) en communication fluidique avec la première chambre de percuteur (223) via l'orifice d'entrée/sortie de gaz (223a) de ladite première chambre de percuteur (223).

2. Ensemble selon la revendication 1, **caractérisé en ce qu'**une paroi de séparation (13) est agencée axialement dans le volume interne (12) de l'enveloppe périphérique (11) et divise ledit volume interne (12) en un premier et un second compartiment (121, 122), les premier et second compartiments (121, 122) contenant ledit au moins un matériau adsorbant (5), et ladite paroi de séparation (13) comprenant au moins un passage de gaz (131) mettant en communication fluidique les premier et second compartiments (121, 122).

3. Ensemble selon la revendication 1, **caractérisé en ce que** le corps principal (21) forme manchon autour d'une partie du récipient (1).

4. Ensemble selon la revendication 1, **caractérisé en ce que** le premier orifice (23) du corps principal (21) est en communication fluidique avec un réservoir flexible (24).

5. Ensemble selon la revendication 1, **caractérisé en ce que** les moyens de perçage (226, 227, 228) comprennent des pointes creuses comprenant des canaux internes (29), les canaux internes (29) des pointes creuses étant en communication fluidique avec les première et deuxième chambres de percuteur (223, 224).

6. Ensemble selon la revendication 5, **caractérisé en ce que** les pointes creuses des moyens de perçage (226, 227, 228) comprennent au moins une pointe creuse additionnelle (227) ayant une longueur supérieure à la longueur de la majorité des autres pointes creuses (226, 228) et comprenant un canal interne (229), ladite pointe creuse additionnelle (227) étant en communication fluidique avec la deuxième chambre de percuteur (224), via le canal interne (229).

7. Ensemble selon la revendication 1, **caractérisé en ce que** le mécanisme d'activation (3) comprend un organe de manipulation (330) actionnable par l'utilisateur coopérant avec une tige filetée (331) venant appuyer sur le percuteur (22) mobile du mécanisme d'amorçage (2).

8. Ensemble selon la revendication 1, **caractérisé en ce que** le volume interne (12) du récipient de gaz (1) contient de l'oxygène à une pression inférieure ou égale à 10 bar absolus.

9. Ensemble selon la revendication 1, **caractérisé en ce qu'**après perçage de l'enveloppe périphérique (11) du récipient de gaz (1) par les moyens de perçage (226, 227, 228) du mécanisme d'amorçage (2), le premier orifice (23) du corps principal (21) est en communication fluidique avec la deuxième chambre de percuteur (224) via l'orifice d'entrée/sortie de gaz (224a) de ladite deuxième chambre de percuteur (224).

10. Ensemble selon la revendication 1, **caractérisé en ce que** le second orifice (25) du corps principal (21) du mécanisme d'activation (3) est en communication fluidique avec une interface respiratoire (30).

## Patentansprüche

1. Gasversorgungsanordnung (1, 4) umfassend einen Gasbehälter (1) und ein Gasentnahmesystem (4), das mit dem Gasbehälter (1) zusammenwirkt, wobei:
- der Gasbehälter (1) eine Umfangshülle (11) umfasst, die ein Innenvolumen (12) definiert, das wenigstens ein Adsorptionsmaterial (5) enthält, und
- das Gasentnahmesystem (4) Folgendes umfasst:
a) einen beweglichen Vorbereitungsmechanismus (2), der Durchbohrungsmittel (226, 227, 228) umfasst, und
b) einen Aktivierungsmechanismus (3), der von einem Benutzer betätigbar ist und mit dem Vorbereitungsmechanismus (2) zusammenwirkt, um den Vorbereitungsmechanismus (2) in Richtung des Gasbehälters (1) zu bewegen, damit die Durchbohrungsmittel (226, 227, 228) des Vorbereitungsmechanismus (2) die Umfangshülle (11) des Gasbehälters (1) durchstechen,
**dadurch gekennzeichnet, dass**
der Aktivierungsmechanismus (3) des Gasentnahmesystems (4) Folgendes umfasst:
- einen Hauptkörper (21), in dem der Vorbereitungsmechanismus (2) angeordnet ist, wobei der Hauptkörper (21) eine erste (23) und eine zweite Öffnung (25) umfasst, und
- eine bewegliche Stoßvorrichtung (22), die eine erste und eine zweite Stoßvorrichtungskammer (223, 224) umfasst, die jeweils eine Ein-/Austrittsöffnung für Gas (223a, 224a) umfassen, wobei die Stoßvorrichtung (22) eine Wandverlängerung (225) aufweist, die dazu ausgebildet ist:
1) die zweite Öffnung (25) des Hauptkörpers (21) des Aktivierungsmechanismus (3) vor dem Durchbohren der Umfangshülle (11) des Gasbehälters (1) durch die Durchbohrungsmittel (226, 227, 228) des Vorbereitungsmechanismus (2) zu verschließen, und
2) die zweite Öffnung (25) des Hauptkörpers (21) des Aktivierungsmechanismus (3) nach dem Durchbohren der Umfangshülle (11) des Gasbehälters (1) durch die Durchbohrungsmittel (226, 227, 228) des Vorbereitungsmechanismus (2) freizugeben und die zweite Öffnung (25) des Hauptkörpers (21) mit der ersten Stoßvorrichtungskammer (223) über die Ein-/Austrittsöffnung für Gas (223a) der ersten Stoßvorrichtungskammer (223) in Fluidverbindung zu bringen.

2. Anordnung nach Anspruch 1, **dadurch gekennzeichnet, dass** eine Trennwand (13) axial im Innenvolumen (12) der Umfangshülle (11) angeordnet ist und das Innenvolumen (12) in einen ersten und einen zweiten Raum (121, 122) teilt, wobei der erste und zweite Raum (121, 122) das wenigstens eine Adsorptionsmaterial (5) enthalten und die Trennwand (13) wenigstens einen Gasdurchgang (131) umfasst, der den ersten und zweiten Raum (121, 122) in Fluidverbindung bringt.

3. Anordnung nach Anspruch 1, **dadurch gekennzeichnet, dass** der Hauptkörper (21) eine Hülse um einen Abschnitt des Behälters (1) bildet.

4. Anordnung nach Anspruch 1, **dadurch gekennzeichnet, dass** die erste Öffnung (23) des Hauptkörpers (21) mit einem flexiblen Vorratsbehälter (24) in Fluidverbindung steht.

5. Anordnung nach Anspruch 1, **dadurch gekennzeichnet, dass** die Durchbohrungsmittel (226, 227, 228) Hohlspitzen umfassen, die Innenkanäle (29) umfassen, wobei die Innenkanäle (29) der Hohlspitzen mit der ersten und zweiten Stoßvorrichtungskammer (223, 224) in Fluidverbindung stehen.

6. Anordnung nach Anspruch 5, **dadurch gekennzeichnet, dass** die Hohlspitzen der Durchbohrungsmittel (226, 227, 228) wenigstens eine Zusatzhohlspitze (227) mit einer Länge größer als die Länge der Mehrheit der anderen Hohlspitzen (226, 228) und einem Innenkanal (229) umfassen, wobei die Zusatzhohlspitze (227) über den Innenkanal (229) mit der zweiten Stoßvorrichtungskammer (224) in Fluidverbindung steht.

7. Anordnung nach Anspruch 1, **dadurch gekennzeichnet, dass** der Aktivierungsmechanismus (3) ein vom Benutzer betätigbares Bedienorgan (330) umfasst, das mit einer Gewindestange (331) zusammenwirkt, die auf die bewegliche Stoßvorrichtung (22) des Vorbereitungsmechanismus (2) drückt.

8. Anordnung nach Anspruch 1, **dadurch gekennzeichnet, dass** das Innenvolumen (12) des Gasbehälters (1) Sauerstoff mit einem Druck kleiner als oder gleich 10 bar absolut enthält.

9. Anordnung nach Anspruch 1, **dadurch gekennzeichnet, dass** nach dem Durchbohren der Umfangshülle (11) des Gasbehälters (1) durch die Durchbohrungsmittel (226, 227, 228) des Vorbereitungsmechanismus (2) die erste Öffnung (23) des Hauptkörpers (21) mit der zweiten Stoßvorrichtungskammer (224) über die Ein-/Austrittsöffnung für Gas (224a) der zweiten Stoßvorrichtungskammer (224) in Fluidverbindung steht.

10. Anordnung nach Anspruch 1, **dadurch gekennzeichnet, dass** die zweite Öffnung (25) des Hauptkörpers (21) des Aktivierungsmechanismus (3) mit einer Beatmungsschnittstelle (30) in Fluidverbindung steht.

## Claims

1. Gas supply assembly (1, 4) comprising a gas container (1) and a gas withdrawing system (4) cooperating with the gas container (1), wherein:
- the gas container (1) comprises a peripheral shell (11) defining an internal volume (12) containing at least one adsorbent material (5), and
- the gas withdrawing system (4) comprises:
a) a movable priming mechanism (2) comprising piercing means (226, 227, 228), and
b)an activation mechanism (3) that is able to be actuated by a user and cooperates with the priming mechanism (2) to move the priming mechanism (2) in the direction of the gas container (1) such that the piercing means (226, 227, 228) of the priming mechanism (2) perforate the peripheral shell (11) of the gas container (1),
**characterized in that**
the activation mechanism (3) of the gas withdrawing system (4) comprises:
- a main body (21) in which the priming mechanism (2) is arranged, said main body (21) comprising a first orifice (23) and a second orifice (25), and
- a movable striker (22) comprising a first and a second striker chamber (223, 224) that each comprise a gas inlet/outlet orifice (223a, 224a), said striker (22) comprising a wall extension (225) configured to:
1) shut off the second orifice (25) in the main body (21) of the activation mechanism (3) before the peripheral shell (11) of the gas container (1) is pierced by the piercing means (226, 227, 228) of the priming mechanism (2), and
2) open up the second orifice (25) in the main body (21) of the activation mechanism (3) after the peripheral shell (11) of the gas container (1) has been pierced by the piercing means (226, 227, 228) of the priming mechanism (2) and place the second orifice (25) of the main body (21) in fluidic communication with the first striker chamber (223) via the gas inlet/outlet orifice (223a) of said first striker chamber (223).

2. Assembly according to Claim 1, **characterized in that** a separating wall (13) is arranged axially in the internal volume (12) of the peripheral shell (11) and divides said internal volume (12) into a first and a second compartment (121, 122), the first and second compartments (121, 122) containing said at least one adsorbent material (5), and said separating wall (13) comprising at least one gas passage (131) placing the first and second compartments (121, 122) in fluidic communication.

3. Assembly according to Claim 1, **characterized in that** the main body (21) forms a sleeve around a part of the container (1).

4. Assembly according to Claim 1, **characterized in that** the first orifice (23) in the main body (21) is in fluidic communication with a flexible reservoir (24).

5. Assembly according to Claim 1, **characterized in that** the piercing means (226, 227, 228) comprise hollow spikes comprising internal channels (29), the internal channels (29) of the hollow spikes being in fluidic communication with the first and second striker chambers (223, 224).

6. Assembly according to Claim 5, **characterized in that** the hollow spikes of the piercing means (226, 227, 228) comprise at least one additional hollow spike (227) having a length greater than the length of the majority of the other hollow spikes (226, 228) and comprising an internal channel (229), said additional hollow spike (227) being in fluidic communication with the second striker chamber (224), via the internal channel (229).

7. Assembly according to Claim 1, **characterized in that** the activation mechanism (3) comprises a manipulation member (330) that is able to be actuated by the user, cooperating with a threaded rod (331) that bears on the movable striker (22) of the priming mechanism (2).

8. Assembly according to Claim 1, **characterized in that** the internal volume (12) of the gas container (1) contains oxygen at a pressure lower than or equal to 10 bar absolute.

9. Assembly according to Claim 1, **characterized in that**, after the peripheral shell (11) of the gas container (1) has been pierced by the piercing means (226, 227, 228) of the priming mechanism (2), the first orifice (23) of the main body (21) is in fluidic communication with the second striker chamber (224) via the gas inlet/outlet orifice (224a) of said second striker chamber (224).

10. Assembly according to Claim 1, **characterized in that** the second orifice (25) of the main body (21) of the activation mechanism (3) is in fluidic communication with a respiratory interface (30).
